(19) **Europäisches Patentamt**
**European Patent Office**
**Office européen des brevets**

(11) **EP 4 167 848 B1**

(12) **EUROPEAN PATENT SPECIFICATION**

(45) Date of publication and mention
of the grant of the patent:
**30.04.2025 Bulletin 2025/18**

(21) Application number: **21737773.8**

(22) Date of filing: **14.06.2021**

(51) International Patent Classification (IPC):
*A61B 5/08* (2006.01)   *A61B 5/083* (2006.01)
*A61B 5/00* (2006.01)   *A61B 5/113* (2006.01)

(52) Cooperative Patent Classification (CPC):
**A61B 5/0816; A61B 5/0833; A61B 5/0836;**
**A61B 5/1135; A61B 5/6831;** A61B 5/024

(86) International application number:
**PCT/IB2021/055207**

(87) International publication number:
**WO 2021/255616 (23.12.2021 Gazette 2021/51)**

(54) **METHOD FOR PROVIDING REAL-TIME INFORMATION ABOUT THE CARDIAC AND/OR RESPIRATORY PERFORMANCE OF AN INDIVIDUAL, AND CORRESPONDING DEVICE**

VERFAHREN ZUR BEREITSTELLUNG VON ECHTZEITINFORMATIONEN ÜBER DIE HERZ- UND/ODER ATEMLEISTUNG EINER PERSON UND ENTSPRECHENDE VORRICHTUNG

PROCÉDÉ DE FOURNITURE D'INFORMATIONS EN TEMPS RÉEL CONCERNANT LES PERFORMANCES CARDIAQUES ET/OU RESPIRATOIRES D'UN INDIVIDU, ET DISPOSITIF CORRESPONDANT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.06.2020 IT 202000014500**

(43) Date of publication of application:
**26.04.2023 Bulletin 2023/17**

(73) Proprietor: **Di Paco, Adriano**
**57128 Livorno (LI) (IT)**

(72) Inventors:
• **RIBA, Andrea**
**10064 Pinerolo (TO) (IT)**
• **TARANTINO, Sergio**
**56123 Pisa (IT)**

• **CECIO, Davide**
**57128 Livorno (IT)**
• **DI PACO, Adriano**
**57128 Livorno (IT)**

(74) Representative: **Metroconsult Srl**
**Via Sestriere, 100**
**10060 None (TO) (IT)**

(56) References cited:
**EP-A1- 3 643 227**     **EP-A2- 3 318 179**
**US-A1- 2004 186 390**     **US-A1- 2017 231 576**

• **T. BELTRAME ET AL: "Prediction of oxygen uptake dynamics by machine learning analysis of wearable sensors during activities of daily living", SCIENTIFIC REPORTS, vol. 7, no. 1, 1 May 2017 (2017-05-01), XP055767896, DOI: 10.1038/srep45738**

**Description**

The present invention relates to a method for providing real-time information about the cardiac and/or respiratory performance of an individual, as well as to an associated device.

It is known that an individual's aerobic power is normally defined in terms of maximum oxygen consumption: such parameter is also referred to as $VO_2$max. The $VO_2$max parameter is used, for example, in order to monitor cardiorespiratory adaptations to aerobic training programs, prescribe exercise intensity for aerobic training, and predict performance levels during resistance-based sports events.

It is also known that a direct measurement of an individual's expired gas samples provides highly reliable and accurate data, and is therefore considered to be the "gold standard", i.e. the most accurate diagnostic test for determining the $VO_2$max parameter of an individual.

In particular, the gold standard used for detecting the $VO_2$max parameter, among others, is undoubtedly the cardiopulmonary stress test.

Very often, however, this test is only available for selected individuals, in particular professional athletes or patients affected by particular disorders, because it requires costly equipment and qualified personnel.

As a consequence, many equations have been developed for estimating the $VO_2$max parameter, which utilize different variables such as, for example, sex, age, height, weight, time of execution of a walk or a run on a treadmill and power thus generated, and so forth.

Such equations have been defined for various reference populations, including for instance men, women, teenager athletes, university students, elderly people, etc. One example of such equations can be found in the article by Malek et al. "A new non-exercise-based VO2max prediction equation for aerobically trained men", Journal of Strength and Conditioning Research, 2005, 19(3), pp. 559-565. Such equations are necessarily inaccurate because, of course, they cannot precisely measure the $VO_2$max parameter of every single individual. Instead, they estimate it on the basis of rough hypotheses assigning the individual to a given reference population.

Wearable garments are also commercially available, in particular chest belts, which are used, in particular, by people practising sports, whether at amateur or professional level, and measure a person's heart rate.

Such garments derive the ventilatory and metabolic parameter ($VO_2$max) from the heart rate. However, they do not provide reliable data, in comparison with the "gold standard" of the cardiopulmonary stress test.

More in detail, in the sports world many resources have been dedicated by professionals, in particular sport physicians and scientists, to evaluating the various aspects of physical response to exercise, with particular reference to muscular force, power, maximum achievable speed and aerobic and anaerobic effort phases; not much attention has been paid to ventilatory response to exercise.

In many studies, cardiorespiratory response to exercise has been used essentially for finding the $VO_2$max and anaerobic threshold values.

Training systems have also evolved over time with the advent of GPS, but they are still essentially based solely on cardiac response to exercise.

For example, metabolic power (postulated by comparing the sprint of a soccer player to an uphill run) extrapolates from the GPS data the athlete's energetic expenditure (GPEXE); or chest belts estimate, through suitable software, the $VO_2$max parameter from the heart rate: see, in this regard, the article by Prampero et al. entitled "Sprint running: a new energetic approach", The Journal of Experimental Biology, August 2005, pp. 2809-2816.

An article available on the Internet webpage https://www.firstbeat.com/wp-content/uploads/2015/10/white_paper_vo2_estimation.pdf suggests the inaccuracy of the respiratory data obtained from chest belts, since it cannot be measured directly.

One of the inventors of the present patent application has executed approximately 2,500 cardiopulmonary stress tests on national and international professional athletes, publishing two scientific papers highlighting that ventilatory response and efficiency to exercise are ineluctable elements for the evaluation of the athletic performance of such athletes (see articles by Di Paco et al, "Changes in ventilatory response to exercise in trained athletes: respiratory physiological benefits beyond cardiovascular performance", Archivos de Bronconéumologìa, February 2017, pp. 237-244; Di Paco et al., "Ventilatory response to exercise of elite soccer players", Multidisciplinary Respiratory Medicine, April 2014, 9:20).

Document EP 3643227 A1 relates to a method for providing an approximation of a minimum heart rate from collected heart rate data of a user. The method comprises calculating, from heartbeat signal collected from a user, a heart rate value and an artefact percentage for one or more time periods of the collected heartbeat signal and qualifying data of the time period(s) for which the user is verified to be awake and immobile, and for which the artefact percentage is under a predetermined value. Data of the other time periods is disqualified. The method further comprises calculating heart rate parameters from the qualified data and applying a function to the heart rate parameters in order to obtain the approximation of minimum heart rate.

Document US 2004/186390 A1 describes a respiratory analyzer comprising a flow module, having a flow tube through which the flow rate of gases is determined using a flow rate meter, and a computation module, in communication with the flow module, operable to determine a flow rate of respired gases. The computation module may also be operable to determine a metabolic rate of the subject.

Document US 2017/231576 A1 describes a filter to cut at least a harmonic component of a frequency component derived from breathing of a person to be observed which is applied to a detected signal of a wireless sensor, and a heartbeat component of the person is detected in a signal having passed through the filter.

Document EP 3318197 A2 relates to a method for simultaneously measuring a respiration rate and a heart rate using dual cameras of a smartphone. The method includes: collecting a PPG signal and visual data on chest and abdomen movements of a human; converting the visual data to an RGBA signal; applying a bandpass filter such that a frequency only within a range in the RGBA signal pass; removing first and last predetermined portions of the RGBA signal to which the band-pass filter is applied; selecting a color channel having a highest standard deviation from the RGBA signal where the predetermined portions are removed; applying a spline filter to the selected color channel; computing power spectrum density; and computing a heart rate and a respiration rate.

The article in the name of T. Beltrame et al. entitled "Prediction of oxygen uptake dynamics by machine learning analysis of wearable sensors during activities of daily living", Scientific Reports, Vol. 7, no. 1, 1 May 2017, develops and verifies methods required to predict and investigate the VO2 dynamics during activities of daily living. Variables derived from wearable sensors are used to create a VO2 predictor based on a random forest method.

It is therefore one object of the present invention to provide a method for providing real-time information about the cardiac and/or respiratory performance of an individual, as well as an associated device.

It is a further object of the present invention to provide a method for providing real-time information about the cardiac and/or respiratory performance of an individual, which is suitable for professional and amateur athletes and also for simple sports enthusiasts.

In brief, the present invention envisages a first embodiment comprising a method divided into two phases.

During a first phase, a subject undergoes a cardiopulmonary stress test in accordance with a given protocol, e.g. on a treadmill, and using a standard protocol (starting at 8 km/h and increasing the speed by 1 km/h per minute until physical exhaustion), for directly measuring the expired gases and volumes. Subsequently, a plurality of characteristic curves are obtained, which are mathematical functions that correlate the ventilatory and metabolic parameters of the individual with the respiratory rate only.

During a second phase of the method, such functions, which are univocally associated with the subject who has undergone the cardiopulmonary stress test, are used in an algorithm, e.g. implemented in an electronic device like, for example, a wearable device such as a smartwatch or a smartphone, or a tablet, an iPad, or even in cloud, for monitoring in real time the respiratory parameters of the individual and comparing them with his/her personal data obtained during the cardiopulmonary stress test, and then providing physiological indications, recommending physical exercises, predicting performance levels, and so forth.

This embodiment of the invention has been specially conceived for professional athletes (soccer players, cyclists, walkers, long-distance runners, etc.), who are regularly subjected to cardiopulmonary stress tests to obtain the authorization for competitive sport activities.

A second embodiment of the invention has been specially devised for non-professional and amateur athletes as well as simple sports enthusiasts.

More in detail, an individual who cannot or does not want to undergo a cardiopulmonary stress test, which, as aforesaid, is expensive and requires properly trained personnel to be executed, can nevertheless advantageously benefit from the results obtained from a plurality of other individuals who have carried out the cardiopulmonary stress test.

In fact, the data obtained from people who have undergone a cardiopulmonary stress test are collected into a special database.

An individual who cannot or does not want to undergo a cardiopulmonary stress test will have to wear a wearable garment, in particular a chest belt, and will repeat the same protocol, e.g. on a treadmill, as that which is used by people who carry out a cardiopulmonary stress test (e.g. starting at 8 km/h and increasing the speed by 1 km/h per minute until physical exhaustion); by measuring just the respiratory rate, the characteristic curves of a subject will be identified, among all the characteristic curves of people who underwent the cardiopulmonary stress test, which approximate most closely the characteristic curves of the individual. This search will be assisted by using anthropometric data of the various subjects, in particular sex, height, weight and age, in order to create some sort of initial division of the same into different groups. Once the reference characteristic curves have been identified, they will be implemented in memory means of the chest belt worn by the non-professional, amateur or enthusiast athlete for monitoring in real time his/her performance, recommending exercises, etc.

A substantial advantage of the method according to the invention lies in the fact that the functions developed by means of the gold standard provide an accurate profile in terms of ventilatory and metabolic cardiac response, whereas with only the heart rate and respiratory rate it would not be possible to extrapolate the aspect related to ventilation and to the metabolic component.

Further advantageous features of the present invention will be set out in the appended claims.

Further features and advantages of the present invention will become more apparent from the following description of an embodiment thereof as shown in the annexed drawings, which are supplied merely by way of non-limiting example, wherein:

- Figure 1 shows a flow chart of a method for providing real-time information about the cardiac and/or respiratory performance of an individual compared with personal data;
- Figure 2 illustrates a table showing an extract of values measured during a cardiopulmonary stress test;
- Figures 3 and 4 show graphs comprising measured values and values obtained by means of a formula according to the present invention, concerning the ventilation and $VO_2$ parameters, respectively;
- Figure 5 shows a block diagram of a wearable garment implementing characteristic curves of cardiac and/or respiratory parameters according to the invention;
- Figure 6 shows a flow chart of a method for providing real-time information about the cardiac and/or respiratory performance of an individual compared with personal data of subjects who have already undergone a cardiopulmonary stress test.

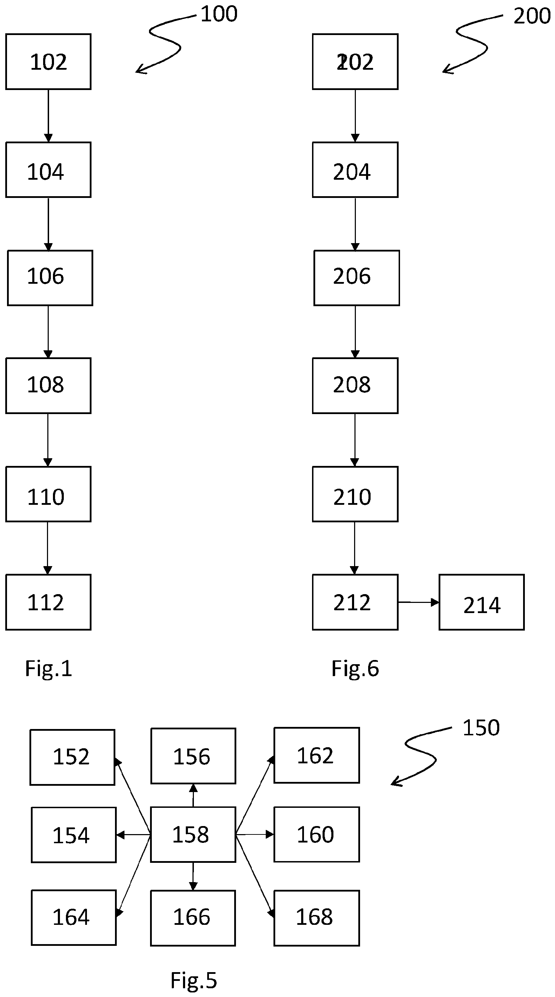

With reference to Figure 1, there is shown a flow chart 100 of a method for providing real-time information about the cardiac and/or respiratory performance of an individual compared with personal data of the same individual formerly obtained.

At step 102, a subject starts a cardiopulmonary stress test in accordance with a given protocol, e.g. on a treadmill using a standard protocol (starting at 8 km/h and increasing the speed by 1 km/h per minute until physical exhaustion). Of course, the test may be carried out by using different means, e.g. a bicycle ergometer, and by following protocols other than said standard protocol.

The subject is connected to an electrocardiograph and, by means of a mouthpiece or a mask, to a breath detector that measures the air volumes taken in at each respiratory action, and to an analyzer that analyzes the gases (carbon dioxide and oxygen) that are exchanged at alveolus level. All the data collected during the exercise are processed by dedicated software, which provides a set of characteristic curves representing the individual adaptation to exercise in terms of ventilation and metabolism.

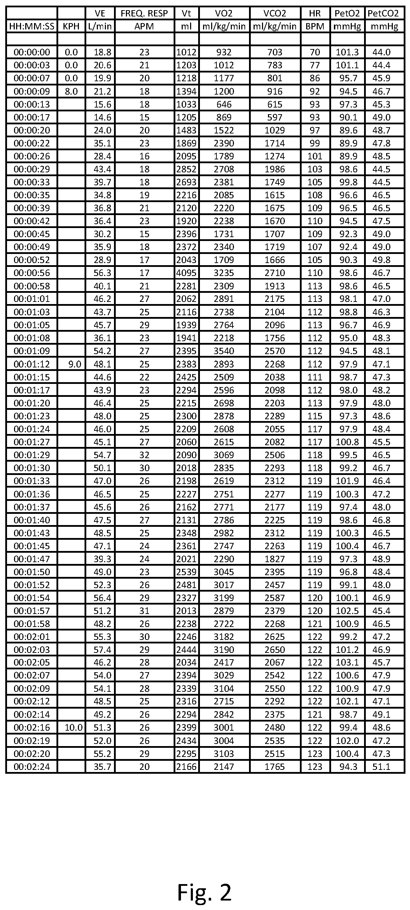

At step 104, a suitable apparatus known in the art directly measures a number of cardiac or respiratory parameters, an extract of which is shown by way of example in Figure 2.

The parameters that are typically measured during a cardiopulmonary stress test are:

"HH:MM": time, measured in hours, minutes and seconds, elapsed from the start of the cardiopulmonary stress test;
"KPH": treadmill speed, expressed in Km/h;
"VE": ventilation, measured in l/min;
"FREQ. RESP": respiratory rate, measured in apm (actions per minute);
"Vt": current volume, expressed in ml;
"$VO_2$": maximum oxygen consumption, expressed in ml/kg/min;
"$VCO_2$": maximum carbon dioxide consumption, expressed in ml/kg/min;
"HR": heart rate, expressed in bpm (beats per minute);
"$PetO_2$": end-expiratory partial oxygen pressure, expressed in mmHg;
"$PetCO_2$": end-expiratory partial carbon dioxide pressure, expressed in mmHg.

At step 106, the individual ends the cardiopulmonary stress test.

At step 108, these parameters are recorded into a database.

At step 110, by using the values entered into the database, one or more characteristic curves, or mathematical functions, are calculated which include only the respiratory rate as dependent variable and, as independent variables, ventilation, current volume, maximum oxygen consumption, maximum carbon dioxide consumption, end-expiratory partial oxygen pressure and end-expiratory partial carbon dioxide pressure.

Such functions are, preferably, second-degree polynomial functions obtained through a second-degree polynomial fitting of the measured values.

Such functions make it possible to obtain the subject's ventilatory and metabolic parameters starting from the subject's respiratory rate alone.

By way of example, ventilation VE can be obtained through the parameters a, b, c obtained from the respiratory rate FREQ.RESP alone, by applying the data obtained from the cardiopulmonary stress test to the formula:

$$VE = a \cdot (FREQ.RESP)^2 + b \cdot (FREQ.RESP) + c \quad (1)$$

where a, b, c are unknown parameters that characterize the characteristic curve of ventilation VE.

By so doing, a single ventilation value will be obtained for a given respiratory rate, such value representing a sort of mean ventilation value of all values detected in the subject during the cardiopulmonary stress test.

In another embodiment, such functions are, preferably, third-degree polynomial functions obtained through a third-degree polynomial fitting of the measured values.

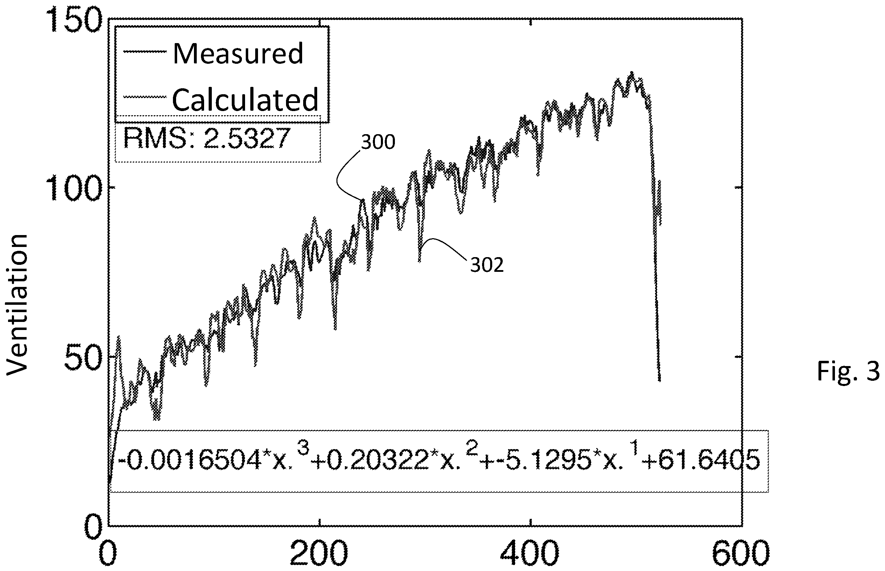

By way of example, as shown in Figure 3, ventilation VE can be obtained through the parameters a, b, c, d obtained from the respiratory rate FREQ.RESP, by applying the data obtained from the cardiopulmonary stress test to the formula:

$$VE = a \cdot (FREQ.RESP)^3 + b \cdot (FREQ.RESP)^2 + c \cdot (FREQ.RESP) + d \quad (2)$$

In the example shown herein, the curve 300 showing an increasing trend with moderate peaks is the curve measured during the cardiopulmonary stress test, while the curve 302 showing more pronounced peaks is the one approximated by the cubic function represented in the Figure by using the formula (2).

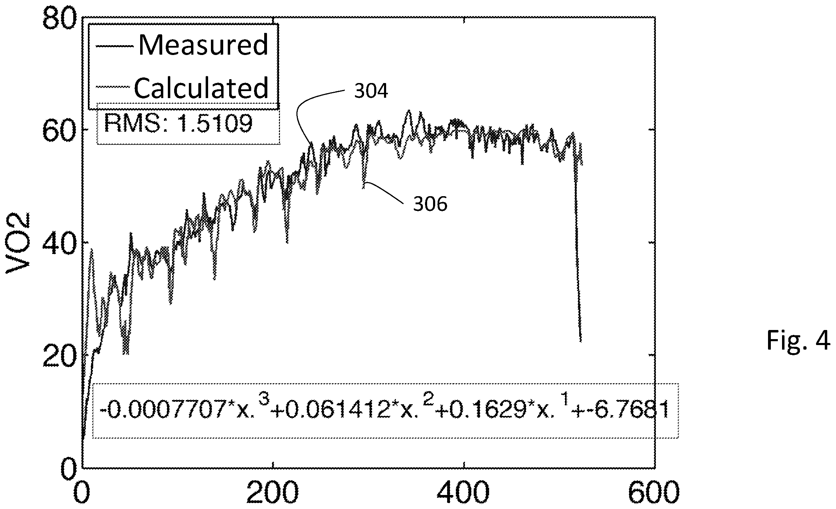

Still by way of example, as shown in Figure 4, the maximum oxygen consumption $VO_2$ can be obtained through the parameters a, b, c, d obtained from the respiratory rate FREQ.RESP, by applying the data obtained from the cardiopulmonary stress test to the formula:

$$VO_2 = a \cdot (FREQ.RESP)^3 + b \cdot (FREQ.RESP)^2 + c \cdot (FREQ.RESP) + d \quad (3)$$

In the example shown herein, the curve 304 showing an increasing trend with moderate peaks is the curve measured during the cardiopulmonary stress test, while the curve 306 showing more pronounced peaks is the one approximated by the cubic function represented in the Figure by using the formula (3).

Referring back to Figure 1, at step 112 the characteristic curves, or mathematical functions, obtained at step 110 are used in an algorithm, e.g. implemented in an electronic device like, for example, a wearable device such as a smartwatch or a smartphone, or a tablet, an iPad, or a wearable garment comprising memory means, or even in cloud, for monitoring in real time the respiratory parameters of the individual and comparing them with his/her personal data obtained during the cardiopulmonary stress test, and then providing physiological indications, recommending physical exercises, predicting performance levels, and so forth.

The electronic device generally comprises a display, and possibly a graphic interface, for providing visual indications to the user. It may also comprise audible signalling means for providing the user with other types of messages and/or alarms and/or indications.

With reference to Figure 5, there is shown a block diagram of a wearable garment 150 according to the present invention.

In order to monitor in real time the athletic performance of a subject, in particular an athlete, he/she will have to wear the wearable garment 150 comprising means 152 for measuring at least his/her respiratory rate.

Preferably, the wearable device is a chest belt.

Even more preferably, the wearable device 150 is a chest belt that measures the subject's respiratory rate via means adapted to detect the extension of the material, e.g. a fabric, it is made of. Chest belts of this kind are known, for example, from patent documents no. CN204133468U, WO2018037855 and EP2578141.

The wearable garment 150 further comprises at least:

- power supply means 154, in particular a Li-Po battery;
- an integrated circuit 156, in particular a dedicated ASIC;
- a microcontroller 158;
- a data transceiving module 160, in particular of the wireless or bluetooth type;
- memory means 162, for implementing the characteristic curves, or mathematical formulas, obtained at step 110.

Typically, such characteristic curves are transmitted to the wearable garment 150 from the database via the data transceiving module 160.

The wearable device may further comprise one or more of the following modules:

- a module 164 for detecting GPS signals;
- a module 166 adapted to detect the heart rate;
- a module 168 adapted to filter the detected cardiac or respiratory signal.

By measuring only the respiratory rate via the wearable garment 150, the subject, in particular an athlete, will be able to monitor, via his/her heart rate, for example, ventilation and maximum oxygen consumption, as well as the evolution of his/her training path, observing in real time his/her improvements (or regressions) as a function of the trend of such current values.

For example, the effort being equal, the subject will be able to compare the values and define a strategy to improve a (ventilatory/metabolic or cardiac) quality and calibrate his/her work based on the accuracy bound to the ventilatory and metabolic datum, i.e. maximum oxygen consumption.

With reference to Figure 6, there is shown a flow chart 200 of a method for providing real-time information about the cardiac and/or respiratory performance of an individual compared with personal data of subjects who have already undergone a cardiopulmonary stress test following the same steps 102, 104, 106, 108 and 110 illustrated herein with reference to the method of Figure 1. In this second embodiment, the database contains values derived from cardiopulmonary stress tests carried out by a plurality of subjects. For the purposes of the method of the invention, the larger the number of tests stored in the database, the greater the effectiveness of the method.

This second embodiment of the method according to the invention has been specially devised for non-professional and amateur athletes as well as simple sports enthusiasts.

At step 202, an individual who cannot or does not want to undergo a cardiopulmonary stress test wears a wearable device comprising means adapted to detect his/her respiratory rate. It is known, in fact, as already mentioned, that for the execution of a cardiopulmonary stress test it is necessary to employ costly equipment and qualified personnel.

At step 204, the individual, by using the same protocol illustrated at step 102, carries out a dynamic test by running on a treadmill or pedalling on a bicycle ergometer or other similar means. It is essential, for the proper application of this embodiment of the method, that the protocol followed by the individual is the same as that which was used by the subjects who underwent the cardiopulmonary stress test according to steps 102, 104, 106, 108 and 110, illustrated herein with reference to the method of Figure 1. Therefore, if the protocol required, for instance, starting at 8 km/h and increasing the speed by 1 km/h per minute until physical exhaustion, the individual will have to replicate the same protocol during the stress test.

At step 206, the respiratory rate detected by the wearable device worn by the individual is stored into suitable memory means of the same device and/or communicated to an external recorder.

The equipment necessary for the stress test of steps 204-206 is advantageously minimal and not very expensive: as a matter of fact, a treadmill (or a bicycle ergometer) and a wearable device detecting the individual's respiratory rate will suffice. Therefore, the test can be carried out without an electrocardiograph, a mask with a mouthpiece, or qualified personnel. The test can even be carried out in a domestic environment, so long as the individual knows the protocol to be followed.

At step 208, the individual ends the motor activity envisaged by the protocol.

At step 210, the respiratory rate detected by the wearable device is transmitted to a cloud, where it is then compared with the values stored in the database, which contains the respiratory values of individuals who have carried out a cardiopulmonary stress test.

At step 212, the functions of a subject are identified, among those contained in the database, which approximate most closely the respiratory values of the individual who has carried out the test at step 202, possibly also taking into account anthropometric data of that subject, in particular sex, height, weight and age.

The functions can be chosen by using statistical techniques (e.g. the least residual or highest verisimilitude techniques) or machine learning techniques (e.g. the K-Means algorithm or neural networks).

At step 214, the curve obtained at step 212 is transmitted to the wearable garment 150 and used in an algorithm, e.g. implemented in an electronic device like, in particular a wearable device such as a smartwatch or a smartphone, or a tablet, an iPad, or even in cloud, for monitoring in real time the current respiratory and/or cardiac parameters of the individual and providing physiological indications, recommending physical exercises, predicting performance, and so forth.

Advantageously, therefore, the individual who has not undergone an expensive cardiopulmonary stress test will be able to benefit from accurate data of subjects who carried out the cardiopulmonary stress test, which is considered to be the gold standard.

The method for providing real-time information about the cardiac and/or respiratory performance of an individual, and the associated device, described herein by way of example may be subject to many possible variations without departing from the novelty spirit of the inventive idea; it is also clear that in the practical implementation of the invention the illustrated details may have different shapes or be replaced with other technically equivalent elements.

For example, the invention also relates to a system comprising a wearable garment 150, in which the individual's characteristic curves are stored, and an electronic device, in particular a smartwatch, a smartphone, a tablet, an iPad or a cloud, which suitably communicates with such wearable garment 150 to provide real-time information about the cardiac and/or respiratory performance of the individual wearing the garment 150.

As an alternative, the system comprises a wearable garment that simply measures the respiratory rate of the individual and communicates with an electronic device worn by the user, in particular a smartwatch or a smartphone, in which the characteristic curves are stored.

It can therefore be easily understood that the present invention is not limited to a method for providing real-time information about the cardiac and/or respiratory performance of an individual and an associated device described herein by way of example, but may be subject to many modifications, improvements or replacements of equivalent parts and elements without departing from the scope of the invention as defined in the following claims.

## Claims

1. Method for providing real-time information about the cardiac and/or respiratory performance of an individual, comprising the steps of:

   a) subjecting said individual to a cardiopulmonary stress test suitable for detecting, over time, a respiratory rate and other cardiac or respiratory parameters of said individual;
   b) determining respective characteristic curves of said individual, with said respiratory rate as dependent variable and one of said other cardiac or respiratory parameters as independent variable;
   c) measuring, through a wearable device, the current respiratory rate of said individual;
   d) determining, based on the current respiratory rate of said individual and said characteristic curves obtained at step b), the other current cardiac or respiratory parameters of said individual;
   e) providing real-time information about the cardiac and/or respiratory performance of said individual on the basis of the other current cardiac or respiratory parameters determined at step d).

2. Method according to claim 1, wherein said other cardiac or respiratory parameters comprise: current volume; maximum oxygen consumption; maximum carbon dioxide consumption; heart rate; end-expiratory partial oxygen pressure; end-expiratory partial carbon dioxide pressure.

3. Method according to claim 1 or 2, wherein said respective characteristic curves of said individual comprise second-degree or third-degree polynomial functions respectively obtained through a second-degree or third-degree polynomial fitting of a respective cardiac or respiratory parameter.

4. Method according to one or more of the preceding claims, wherein said information is made available on a device, in particular a smartwatch, a smartphone, a tablet, an iPad, said wearable device or a cloud.

5. Method according to one or more of the preceding claims, wherein said wearable device comprises a wearable garment (150), in particular a chest belt.

6. Method according to claim 5, wherein said chest belt measures said respiratory rate of the individual through means adapted to detect the extension of the material it is made of.

7. Method for providing real-time information about the cardiac and/or respiratory performance of an individual, comprising the steps of:

   a) subjecting a plurality of subjects to a cardiopulmonary stress test according to a predefined protocol, said cardiopulmonary stress test being adapted to detect, over time, a respiratory rate and other cardiac or respiratory parameters of said plurality of subjects;
   b) for each one of said subjects, determining respective characteristic curves, with said respiratory rate as dependent variable and one of said other cardiac or respiratory parameters as independent variable;
   c) detecting, through a wearable device, in particular a chest belt, a respiratory rate of said individual by subjecting him/her to a pulmonary dynamic test, in particular requiring him/her to run on a treadmill or to pedal on a bicycle ergometer, in accordance with said predefined protocol;
   d) identifying characteristic curves of said individual, said characteristic curves of said individual being, among all the characteristic curves of said subjects, those belonging to a subject which approximate most closely said respiratory rate of said individual;
   e) detecting, through said wearable device, the current respiratory rate of said individual;
   f) determining, based on the current respiratory rate of said individual and said characteristic curves of said individual, the other current cardiac or respiratory parameters of said individual;
   g) providing real-time information about the cardiac and/or respiratory performance of said individual on the basis of the other current cardiac and/or respiratory parameters determined at step f).

8. Method according to claim 7, wherein the identification of the characteristic curves of said individual of step d) is accomplished by using statistical techniques, in particular the least residual or highest verisimilitude ones, or machine learning techniques, in particular the K-Means algorithm or neural networks.

9. Method according to claim 7, wherein said other cardiac or respiratory parameters comprise: current volume; maximum oxygen consumption; maximum carbon dioxide consumption; heart rate; end-expiratory partial oxygen pressure; end-expiratory partial carbon dioxide pressure.

10. Method according to claim 7, wherein said respective first characteristic curves of said individual comprise second-degree or third-degree polynomial functions respectively obtained through a second-degree or third-degree polynomial fitting of a respective cardiac or respiratory parameter.

11. Device (150) wearable by an individual, comprising:

- means (152) for measuring the respiratory rate of said individual to obtain a measured respiration rate of said individual;
- power supply means (154), in particular a Li-Po battery;
- an integrated circuit (156), in particular a dedicated ASIC;
- a microcontroller (158);
- a data transceiving module (160), in particular of the wireless or bluetooth type;
- memory means (162) storing characteristic curves of cardiac and respiratory parameters, said characteristic curves having a respiratory rate as dependent variable and one of other cardiac or respiratory parameters as independent variable and being obtained through a cardiopulmonary stress test, wherein said device (150) is configured to compare said measured respiratory rate of said individual with said characteristic curves for providing real-time information about the cardiac and/or respiratory performance of said individual.

12. Wearable device according to claim 11, wherein said device comprises one or more of the following modules: a module (164) for detecting GPS signals; a module (166) adapted to detect the heart rate; a module (168) adapted to filter the detected signal.

13. Device according to claim 11 or 12, wherein said device comprises a garment, in particular a chest belt.

14. Device according to claim 13, wherein said chest belt is adapted to measure the respiratory rate of said individual through means adapted to detect the extension of the material, in particular a fabric, it is made of.

15. System for providing real-time information about the cardiac and/or respiratory performance of an individual, comprising a device (150) wearable by an individual according to one or more of claims 11 to 14, in particular a chest belt, and an electronic device, said device (150) wearable by said individual being adapted to provide real-time information about the cardiac and/or respiratory performance of the individual to said electronic device.

**Patentansprüche**

1. Verfahren zur Bereitstellung von Echtzeitinformationen über die Herz- und/oder Atmungsleistung einer Person, das die Schritte umfasst:

a) Unterziehen der Person einem kardiopulmonalen Belastungstest, der geeignet ist, im Verlauf der Zeit eine Atmungsfrequenz und andere Herz- oder Atemparameter der Person zu ermitteln;
b) Bestimmen der jeweiligen charakteristischen Kurven der Person, wobei die Atmungsfrequenz eine abhängige Variable und einer der anderen Herz- oder Atemparameter eine unabhängige Variable ist;
c) Messen der aktuellen Atemfrequenz der Person durch ein tragbares Gerät;
d) Bestimmen der anderen aktuellen Herz- oder Atemparameter der Person auf der Grundlage der aktuellen Atemfrequenz der Person und der in Schritt b) erhaltenen charakteristischen Kurven;
e) Bereitstellen von Echtzeitinformationen über die Herz- und/oder Atmungsleistung der Person auf der Grundlage der anderen aktuellen Herz- oder Atemparameter, die in Schritt d) bestimmt wurden.

2. Verfahren nach Anspruch 1, wobei die anderen Herz- oder Atemparameter umfassen: aktuelles Volumen; maximaler Sauerstoffverbrauch; maximaler Kohlendioxidverbrauch; Herzfrequenz; endexspiratorischer Sauerstoffpartialdruck;

endexspiratorischer Kohlendioxidpartialdruck.

3. Verfahren nach Anspruch 1 oder 2, wobei die jeweiligen charakteristischen Kurven der Person Polynomfunktionen zweiten oder dritten Grades umfassen, die jeweils durch eine Polynomanpassung zweiten oder dritten Grades eines jeweiligen Herz- oder Atemparameters erhalten werden.

4. Verfahren nach einem oder mehreren der vorstehenden Ansprüche, wobei die Informationen auf einem Gerät, insbesondere einer Smartwatch, einem Smartphone, einem Tablet, einem iPad, dem tragbaren Gerät oder einer Cloud, verfügbar gemacht werden.

5. Verfahren nach einem oder mehreren der vorstehenden Ansprüche, wobei das tragbare Gerät ein tragbares Kleidungsstück (150), insbesondere einen Brustgurt, umfasst.

6. Verfahren nach Anspruch 5, wobei der Brustgurt die Atemfrequenz der Person durch Mittel misst, die so angepasst sind, dass sie die Ausdehnung des Materials, aus dem er besteht, erfassen.

7. Verfahren zur Bereitstellung von Echtzeitinformationen über die Herz- und/oder Atmungsleistung einer Person, das die folgenden Schritte umfasst:

   a) Unterziehen mehrerer Probanden einem kardiopulmonalen Belastungstest gemäß einem vordefinierten Protokoll, wobei der kardiopulmonale Belastungstest so ausgelegt ist, dass er im Verlauf der Zeit eine Atmungsfrequenz und andere Herz- oder Atemparameter der mehreren Probanden erfasst;
   b) für jede der Personen, Bestimmen jeweiliger charakteristischer Kurven, wobei die Atemfrequenz eine abhängige Variable und einer der anderen Herz- oder Atemparameter eine unabhängige Variable ist;
   c) Erfassen einer Atemfrequenz der Person durch ein tragbares Gerät, insbesondere einen Brustgurt, indem sie gemäß dem vordefinierten Protokoll einem pulmonalen dynamischen Test unterzogen wird, insbesondere indem sie auf einem Laufband laufen oder auf einem Fahrradergometer treten muss;
   d) Identifizieren von charakteristischen Kurven der Person, wobei die charakteristischen Kurven der Person unter allen charakteristischen Kurven der Probanden diejenigen sind, die einem Probanden gehören, die sich der Atemfrequenz der Person am nächsten annähern;
   e) Erfassen der aktuellen Atemfrequenz der Person durch das tragbare Gerät;
   f) Bestimmen der anderen aktuellen Herz- oder Atemparameter der Person auf der Grundlage der aktuellen Atemfrequenz der Person und der charakteristischen Kurven der Person;
   g) Bereitstellen von Echtzeitinformationen über die Herz- und/oder Atmungsleistung der Person auf der Grundlage der anderen aktuellen Herz- und/oder Atemparameter, die in Schritt f) bestimmt wurden.

8. Verfahren nach Anspruch 7, wobei das Identifizieren der charakteristischen Kurven der Einzelperson aus Schritt d) unter Verwendung statistischer Verfahren, insbesondere der Verfahren mit dem geringsten Restfehler oder der höchsten Wahrscheinlichkeit, oder maschineller Lernverfahren, insbesondere des K-Means-Algorithmus oder neuronaler Netze, durchgeführt wird.

9. Verfahren nach Anspruch 7, wobei die anderen Herz- oder Atemparameter umfassen: aktuelles Volumen; maximaler Sauerstoffverbrauch; maximaler Kohlendioxidverbrauch; Herzfrequenz; endexspiratorischer Sauerstoffpartialdruck; endexspiratorischer Kohlendioxidpartialdruck.

10. Verfahren nach Anspruch 7, wobei die jeweiligen ersten charakteristischen Kurven der Person Polynomfunktionen zweiten oder dritten Grades umfassen, die jeweils durch eine Polynomanpassung zweiten oder dritten Grades eines jeweiligen Herz- oder Atemparameters erhalten werden.

11. Von einer Person tragbares Gerät (150), umfassend:

   - Mittel (152) zum Messen der Atemfrequenz der Person, um eine gemessene Atemfrequenz der Person zu erhalten;
   - Stromversorgungsmittel (154), insbesondere eine Li-Po-Batterie;
   - eine integrierte Schaltung (156), insbesondere ein dedizierter ASIC;
   - einen Mikrocontroller (158);
   - ein Daten-Sende-/Empfangsmodul (160), insbesondere vom drahtlosen oder Bluetooth-Typ;
   - Speichermittel (162), die charakteristische Kurven von Herz- und Atemparametern speichern, wobei die charakteristischen Kurven eine Atemfrequenz als eine abhängige Variable und einen von anderen Herz- oder Atemparametern als eine unabhängige Variable aufweisen und durch einen kardiopulmonalen Belastungstest erhalten werden, wobei das Gerät (150) so konfiguriert ist, dass es die gemessene Atemfrequenz der Person mit den charakteristischen Kurven vergleicht, um Echtzeitinformationen über die Herz- und/oder Atmungsleistung der Person bereitzustellen.

12. Tragbares Gerät nach Anspruch 11, wobei das Gerät eines oder mehrere der folgenden Module umfasst: ein Modul (164) zum Erfassen von GPS-Signalen; ein Modul (166), das zum Erfassen der Herzfrequenz ausgelegt ist; ein Modul (168), das zum Filtern des erfassten Signals ausgelegt ist.

13. Gerät nach Anspruch 11 oder 12, wobei das Gerät ein Kleidungsstück umfasst, insbesondere einen Brustgurt.

14. Gerät nach Anspruch 13, wobei der Brustgurt so angepasst ist, dass er die Atemfrequenz der Person durch Mittel misst, die so angepasst sind, dass sie die Ausdehnung des Materials, insbesondere eines Gewebes, aus dem er besteht, erfassen.

15. System zur Bereitstellung von Echtzeitinformationen über die Herz- und/oder Atmungsleistung einer Person, umfassend ein von einer Person tragbares Gerät (150) gemäß einem oder mehreren der Ansprüche 11 bis 14, insbesondere einen Brustgurt, und ein elektronisches Gerät, wobei das von der Person tragbare Gerät (150) so ausgelegt ist, dass es Echtzeitinformationen über die Herz- und/oder Atmungsleistung der Person an das elektronische Gerät liefert.

**Revendications**

1. - Procédé de fourniture d'informations en temps réel concernant les performances cardiaques et/ou respiratoires d'un individu, comprenant les étapes suivantes :

   a) soumettre ledit individu à un test d'effort cardiopulmonaire conçu pour détecter, au cours du temps, une fréquence respiratoire et d'autres paramètres cardiaques ou respiratoires dudit individu ;
   b) déterminer des courbes caractéristiques respectives dudit individu, avec ladite fréquence respiratoire en tant que variable dépendante et l'un desdits autres paramètres cardiaques ou respiratoires en tant que variable indépendante ;
   c) mesurer, par l'intermédiaire d'un dispositif portable, la fréquence respiratoire actuelle dudit individu ;
   d) déterminer, sur la base de la fréquence respiratoire actuelle dudit individu et desdites courbes caractéristiques obtenues à l'étape b), les autres paramètres cardiaques ou respiratoires actuels dudit individu ;
   e) fournir des informations en temps réel concernant les performances cardiaques et/ou respiratoires dudit individu sur la base des autres paramètres cardiaques ou respiratoires actuels déterminés à l'étape d).

2. - Procédé selon la revendication 1, dans lequel lesdits autres paramètres cardiaques ou respiratoires comprennent : le volume actuel ; la consommation maximale d'oxygène ; la consommation maximale de dioxyde de carbone ; la fréquence cardiaque ; la pression partielle d'oxygène en fin d'expiration ; la pression partielle de dioxyde de carbone en fin d'expiration.

3. - Procédé selon la revendication 1 ou 2, dans lequel lesdites courbes caractéristiques respectives dudit individu comprennent des fonctions polynomiales de deuxième degré ou de troisième degré obtenues respectivement par un ajustement polynomial de deuxième degré ou de troisième degré d'un paramètre cardiaque ou respiratoire respectif.

4. - Procédé selon une ou plusieurs des revendications précédentes, dans lequel lesdites informations sont rendues disponibles sur un dispositif, notamment une montre intelligente, un téléphone intelligent, une tablette, un iPad, ledit dispositif portable ou un nuage informatique.

5. - Procédé selon une ou plusieurs des revendications précédentes, dans lequel ledit dispositif portable comprend un vêtement portable (150), en particulier une ceinture thoracique.

6. - Procédé selon la revendication 5, dans lequel ladite ceinture thoracique mesure ladite fréquence respiratoire de l'individu grâce à des moyens conçus pour détecter l'extension du matériau dont elle est constituée.

**7.** - Procédé de fourniture d'informations en temps réel sur les performances cardiaques et/ou respiratoires d'un individu, comprenant les étapes suivantes :

a) soumettre une pluralité de sujets à un test d'effort cardiopulmonaire selon un protocole prédéfini, ledit test d'effort cardiopulmonaire étant conçu pour détecter, au cours du temps, une fréquence respiratoire et d'autres paramètres cardiaques ou respiratoires de ladite pluralité de sujets ;

b) pour chacun desdits sujets, déterminer des courbes caractéristiques respectives, avec ladite fréquence respiratoire en tant que variable dépendante et l'un desdits autres paramètres cardiaques ou respiratoires en tant que variable indépendante ;

c) détecter, par l'intermédiaire d'un dispositif portable, notamment une ceinture thoracique, une fréquence respiratoire dudit individu en le soumettant à un test dynamique pulmonaire, notamment en lui demandant de courir sur un tapis roulant ou de pédaler sur un vélo ergomètre, conformément audit protocole prédéfini ;

d) identifier des courbes caractéristiques dudit individu, lesdites courbes caractéristiques dudit individu étant, parmi toutes les courbes caractéristiques desdits sujets, celles appartenant à un sujet qui se rapprochent le plus de la fréquence respiratoire dudit individu ;

e) détecter, par l'intermédiaire dudit dispositif portable, la fréquence respiratoire actuelle dudit individu ;

f) déterminer, sur la base de la fréquence respiratoire actuelle dudit individu et desdites courbes caractéristiques dudit individu, les autres paramètres cardiaques ou respiratoires actuels dudit individu ;

g) fournir des informations en temps réel concernant les performances cardiaques et/ou respiratoires dudit individu sur la base des autres paramètres cardiaques et/ou respiratoires actuels déterminés à l'étape f).

**8.** - Procédé selon la revendication 7, dans lequel l'identification des courbes caractéristiques dudit individu de l'étape d) est réalisée en utilisant des techniques statistiques, notamment celles de moindre résidu ou de maximum de vraisemblance, ou des techniques d'apprentissage automatique, notamment l'algorithme K-Means ou les réseaux neuronaux.

**9.** - Procédé selon la revendication 7, dans lequel lesdits autres paramètres cardiaques ou respiratoires comprennent : le volume actuel ; la consommation maximale d'oxygène ; la consommation maximale de dioxyde de carbone ; la fréquence cardiaque ; la pression partielle d'oxygène en fin d'expiration ; la pression partielle de dioxyde de carbone en fin d'expiration.

**10.** - Procédé selon la revendication 7, dans lequel lesdites premières courbes caractéristiques respectives dudit individu comprennent des fonctions polynomiales de deuxième degré ou de troisième degré obtenues respectivement par un ajustement polynomial de deuxième degré ou de troisième degré d'un paramètre cardiaque ou respiratoire respectif.

**11.** - Dispositif (150) portable par un individu, comprenant :

- des moyens (152) de mesure de la fréquence respiratoire dudit individu pour obtenir une fréquence respiratoire mesurée dudit individu ;
- des moyens d'alimentation (154), notamment une batterie Li-Po ;
- un circuit intégré (156), notamment un ASIC dédié ;
- un microcontrôleur (158) ;
- un module d'émission-réception de données (160), notamment de type sans fil ou bluetooth ;
- des moyens de mémoire (162) stockant des courbes caractéristiques de paramètres cardiaques et respiratoires, lesdites courbes caractéristiques ayant une fréquence respiratoire en tant que variable dépendante et l'un des autres paramètres cardiaques ou respiratoires en tant que variable indépendante et étant obtenu par l'intermédiaire d'un test d'effort cardiopulmonaire, dans lequel ledit dispositif (150) est conçu pour comparer ladite fréquence respiratoire mesurée dudit individu avec lesdites courbes caractéristiques pour fournir des informations en temps réel concernant les performances cardiaques et/ou respiratoires dudit individu.

**12.** - Dispositif portable selon la revendication 11, dans lequel ledit dispositif comprend un ou plusieurs des modules suivants : un module (164) de détection de signaux GPS ; un module (166) conçu pour détecter la fréquence cardiaque ; un module (168) conçu pour filtrer le signal détecté.

**13.** - Dispositif selon la revendication 11 ou 12, dans lequel ledit dispositif comprend un vêtement, notamment une ceinture thoracique.

**14.** - Dispositif selon la revendication 13, dans lequel ladite ceinture thoracique est conçue pour mesurer la fréquence respiratoire dudit individu par l'intermédiaire de moyens conçus pour détecter l'extension du matériau, notamment un tissu, dont elle est constituée.

15. - Système permettant de fournir des informations en temps réel concernant les performances cardiaques et/ou respiratoires d'un individu, comprenant un dispositif (150) portable par un individu selon l'une ou plusieurs des revendications 11 à 14, notamment une ceinture thoracique, et un dispositif électronique, ledit dispositif (150) portable par ledit individu étant adapté pour fournir des informations en temps réel concernant les performances cardiaques et/ou respiratoires de l'individu audit dispositif électronique.

100

102

104

106

108

110

112

Fig.1

200

202

204

206

208

210

212 → 214

Fig.6

150

152

154

164

156

158

162

160

166

168

Fig.5

| HH:MM:SS | KPH | VE L/min | FREQ. RESP APM | Vt ml | VO2 ml/kg/min | VCO2 ml/kg/min | HR BPM | PetO2 mmHg | PetCO2 mmHg |
|---|---|---|---|---|---|---|---|---|---|
| 00:00:00 | 0.0 | 18.8 | 23 | 1012 | 932 | 703 | 70 | 101.3 | 44.0 |
| 00:00:03 | 0.0 | 20.6 | 21 | 1203 | 1012 | 783 | 77 | 101.1 | 44.4 |
| 00:00:07 | 0.0 | 19.9 | 20 | 1218 | 1177 | 801 | 86 | 95.7 | 45.9 |
| 00:00:09 | 8.0 | 21.2 | 18 | 1394 | 1200 | 916 | 92 | 94.5 | 46.7 |
| 00:00:13 | | 15.6 | 18 | 1033 | 646 | 615 | 93 | 97.3 | 45.3 |
| 00:00:17 | | 14.6 | 15 | 1205 | 869 | 597 | 93 | 90.1 | 49.0 |
| 00:00:20 | | 24.0 | 20 | 1483 | 1522 | 1029 | 97 | 89.6 | 48.7 |
| 00:00:22 | | 35.1 | 23 | 1869 | 2390 | 1714 | 99 | 89.9 | 47.8 |
| 00:00:26 | | 28.4 | 16 | 2095 | 1789 | 1274 | 101 | 89.9 | 48.5 |
| 00:00:29 | | 43.4 | 18 | 2852 | 2708 | 1986 | 103 | 98.6 | 44.5 |
| 00:00:33 | | 39.7 | 18 | 2693 | 2381 | 1749 | 105 | 99.8 | 44.5 |
| 00:00:35 | | 34.8 | 19 | 2216 | 2085 | 1615 | 108 | 96.6 | 46.5 |
| 00:00:39 | | 36.8 | 21 | 2120 | 2220 | 1675 | 109 | 96.5 | 46.5 |
| 00:00:42 | | 36.4 | 23 | 1920 | 2238 | 1670 | 110 | 94.5 | 47.5 |
| 00:00:45 | | 30.2 | 15 | 2396 | 1731 | 1707 | 109 | 92.3 | 49.0 |
| 00:00:49 | | 35.9 | 18 | 2372 | 2340 | 1719 | 107 | 92.4 | 49.0 |
| 00:00:52 | | 28.9 | 17 | 2043 | 1709 | 1666 | 105 | 90.3 | 49.8 |
| 00:00:56 | | 56.3 | 17 | 4095 | 3235 | 2710 | 110 | 98.6 | 46.7 |
| 00:00:58 | | 40.1 | 21 | 2281 | 2309 | 1913 | 113 | 98.6 | 46.5 |
| 00:01:01 | | 46.2 | 27 | 2062 | 2891 | 2175 | 113 | 98.1 | 47.0 |
| 00:01:03 | | 43.7 | 25 | 2116 | 2738 | 2104 | 112 | 98.8 | 46.3 |
| 00:01:05 | | 45.7 | 29 | 1939 | 2764 | 2096 | 113 | 96.7 | 46.9 |
| 00:01:08 | | 36.1 | 23 | 1941 | 2218 | 1756 | 112 | 95.0 | 48.3 |
| 00:01:09 | | 54.2 | 27 | 2395 | 3540 | 2570 | 112 | 94.5 | 48.1 |
| 00:01:12 | 9.0 | 48.1 | 25 | 2383 | 2893 | 2268 | 112 | 97.9 | 47.1 |
| 00:01:15 | | 44.6 | 22 | 2425 | 2509 | 2038 | 111 | 98.7 | 47.3 |
| 00:01:17 | | 43.9 | 23 | 2294 | 2596 | 2098 | 112 | 98.0 | 48.2 |
| 00:01:20 | | 46.4 | 25 | 2215 | 2698 | 2203 | 113 | 97.9 | 48.0 |
| 00:01:23 | | 48.0 | 25 | 2300 | 2878 | 2289 | 115 | 97.3 | 48.6 |
| 00:01:24 | | 46.0 | 25 | 2209 | 2608 | 2055 | 117 | 97.9 | 48.4 |
| 00:01:27 | | 45.1 | 27 | 2060 | 2615 | 2082 | 117 | 100.8 | 45.5 |
| 00:01:29 | | 54.7 | 32 | 2090 | 3069 | 2506 | 118 | 99.5 | 46.5 |
| 00:01:30 | | 50.1 | 30 | 2018 | 2835 | 2293 | 118 | 99.2 | 46.7 |
| 00:01:33 | | 47.0 | 26 | 2198 | 2619 | 2312 | 119 | 101.9 | 46.4 |
| 00:01:36 | | 46.5 | 25 | 2227 | 2751 | 2277 | 119 | 100.3 | 47.2 |
| 00:01:37 | | 45.6 | 26 | 2162 | 2771 | 2177 | 119 | 97.4 | 48.0 |
| 00:01:40 | | 47.5 | 27 | 2131 | 2786 | 2225 | 119 | 98.6 | 46.8 |
| 00:01:43 | | 48.5 | 25 | 2348 | 2982 | 2312 | 119 | 100.3 | 46.5 |
| 00:01:45 | | 47.1 | 24 | 2361 | 2747 | 2263 | 119 | 100.4 | 46.7 |
| 00:01:47 | | 39.3 | 24 | 2021 | 2290 | 1827 | 119 | 97.3 | 48.9 |
| 00:01:50 | | 49.0 | 23 | 2539 | 3045 | 2395 | 119 | 96.8 | 48.4 |
| 00:01:52 | | 52.3 | 26 | 2481 | 3017 | 2457 | 119 | 99.1 | 48.0 |
| 00:01:54 | | 56.4 | 29 | 2327 | 3199 | 2587 | 120 | 100.1 | 46.9 |
| 00:01:57 | | 51.2 | 31 | 2013 | 2879 | 2379 | 120 | 102.5 | 45.4 |
| 00:01:58 | | 48.2 | 26 | 2238 | 2722 | 2268 | 121 | 100.9 | 46.5 |
| 00:02:01 | | 55.3 | 30 | 2246 | 3182 | 2625 | 122 | 99.2 | 47.2 |
| 00:02:03 | | 57.4 | 29 | 2444 | 3190 | 2650 | 122 | 101.2 | 46.9 |
| 00:02:05 | | 46.2 | 28 | 2034 | 2417 | 2067 | 122 | 103.1 | 45.7 |
| 00:02:07 | | 54.0 | 27 | 2394 | 3029 | 2542 | 122 | 100.6 | 47.9 |
| 00:02:09 | | 54.1 | 28 | 2339 | 3104 | 2550 | 122 | 100.9 | 47.9 |
| 00:02:12 | | 48.5 | 25 | 2316 | 2715 | 2292 | 122 | 102.1 | 47.1 |
| 00:02:14 | | 49.2 | 26 | 2294 | 2842 | 2375 | 121 | 98.7 | 49.1 |
| 00:02:16 | 10.0 | 51.3 | 26 | 2399 | 3001 | 2480 | 122 | 99.4 | 48.6 |
| 00:02:19 | | 52.0 | 26 | 2434 | 3004 | 2535 | 122 | 102.0 | 47.2 |
| 00:02:20 | | 55.2 | 29 | 2295 | 3103 | 2515 | 123 | 100.4 | 47.3 |
| 00:02:24 | | 35.7 | 20 | 2166 | 2147 | 1765 | 123 | 94.3 | 51.1 |

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3643227 A1
- US 2004186390 A1
- US 2017231576 A1
- EP 3318197 A2
- CN 204133468 U
- WO 2018037855 A
- EP 2578141 A

### Non-patent literature cited in the description

- **MALEK et al.** A new non-exercise-based VO2max prediction equation for aerobically trained men. *Journal of Strength and Conditioning Research*, 2005, vol. 19 (3), 559-565
- **PRAMPERO et al.** Sprint running: a new energetic approach. *The Journal of Experimental Biology*, August 2005, 2809-2816
- **DI PACO et al.** Changes in ventilatory response to exercise in trained athletes: respiratory physiological benefits beyond cardiovascular performance. *Archivos de Bronconéumologìa*, February 2017, 237-244
- **DI PACO et al.** Ventilatory response to exercise of elite soccer players. *Multidisciplinary Respiratory Medicine*, April 2014, vol. 9, 20
- **T. BELTRAME et al.** Prediction of oxygen uptake dynamics by machine learning analysis of wearable sensors during activities of daily living. *Scientific Reports*, 01 May 2017, vol. 7 (1)